# EUROPEAN PATENT APPLICATION

(11) **EP 4 089 098 A1**
(43) Date of publication of application: **16.11.2022**
(21) Application number: 21172924.9
(22) Date of filing: 10.05.2021
(51) Int. Cl.: C07H 19/10, C07H 19/20, C12Q 1/68

(54) **FRET DYE LABELED REVERSIBLE NUCLEOTIDE TERMINATORS AND THEIR USE IN SEQUENCING OF DNA**

(71) Applicant: Miltenyi Biotec B.V. & Co. KG, 51429 Bergisch Gladbach (DE)
(72) Inventor: MARMA, Mong, 51429 Bergisch Gladbach (DE); GUAN, Xiao-Pei, 51429 Bergisch Gladbach (DE); SOLIMAN, Sameh, 51429 Bergisch Gladbach (DE); PERBOST, Michel, 51429 Bergisch Gladbach (DE)
(74) Representative: Kisters, Michael Marcus

(57) **Abstract**

The invention is directed to a deoxynucleotide triphosphate comprising a FRET energy transfer dye pair according to formula (I) 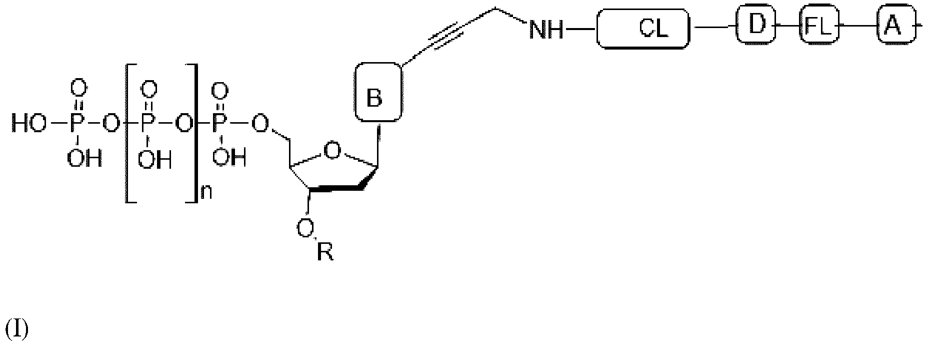 wherein B is a nucleobase
R is a reversible capping group
n is an integer from 1 to 10
CL is a chemically cleavable linker
D is a donor fluorescent dye
A is an acceptor dye
FL is a linker group

## Description

### BACKGROUND

The present invention provides FRET dye labeled reversible nucleotide terminators, in particular where the 3'-OH is capped with chemically removable group, such as -CH₂SSMe, -CH₂N₃ and the FRET dye connected to nucleotides *via* a cleavable linker as well as their use in methods for DNA sequencing. Such compounds provide a new possibility for future sequencing of DNA and RNA molecules, including but not limited to sequencing by synthesis.

A unique advantage of the use this class of reversible terminators is that all four labeled nucleotides could be detected by exciting with a single laser beam. It could bring down the manufacturing cost of the sequencers, and help speed up the sequencing runs.

FRET dye labeled reversible nucleotides can be used in new generation DNA sequencing technologies, often called next-generation DNA sequencing technologies (NGS) where solid surface bound DNA molecules are sequenced by iterative cycle of enzymatic single base incorporation, imaging and cleavage steps.

Next-generation DNA sequencing technology (NGS) is one of the most important tools available in life sciences. It allows high throughput DNA sequencing. This technology is routinely used for genome sequencing, sequencing of multiple samples and genes in a single experiment. In recent years, this technology has been the driving force for the rapid growth and advancement in the field of molecular diagnostics, precision medicines and synthetic biology, etc. Detailed reviews on current NGS technologies are provided by Jerzy K. Kulski, Next-generation sequencing - an overview of the history, tools, and "omic" applications, a book chapter in Next Generation Sequencing - Advances, Applications and Challenges, 2016, M. L. Metzker, Nature Reviews 2010, 11, 31, and C. W. Fuller et al., Nature Biotechnology 2009, 27, 1013.

At the core of the most widely used NGS technology is reversible nucleotide terminators. In standard construct of the reversible nucleotide terminators, the 3'-OH is capped with a reversible group and each of the four bases is labeled by four different fluorescent dyes, attached via a cleavable linker. The nucleobase sequences in DNA is determined by enzymatic single base extension, followed by sequential excitation of the fluorescently labeled nucleotides by four specific wavelengths of lights, and image is taken after each time of excitation by their corresponding specific filter sets. Therefore, the sequencing instrument requires four laser sources, which add up significant cost. Furthermore, in the sequencing runs, this consequently take relatively longer time. In fact, imaging step is one of the slowest steps in overall sequencing cycle.

### SUMMARY

Here we introduce reversible nucleotide terminator labeled with FRET energy transfer dye pairs (Fluorescence Resonance Energy Transfer dyes), which could potentially help overcome above stated limitations. When such reversible terminators are used, next-generation DNA sequencers can be built using only single laser source, because it could potentially allow exciting all four labeled nucleotide bases by a single laser source. It could not only cut the cost of instrument, it could also cut image time by a fourth.

This invention is about the use of reversibly terminating nucleotides labeled with FRET dyes attached to modified nucleotides via cleavable linkers. It can also be in combination with standard, single dye labeled reversibly terminating nucleotides.

Accordingly, a first object of the invention are deoxynucleotide triphosphates comprising a FRET energy transfer dye pair according to formula (I) 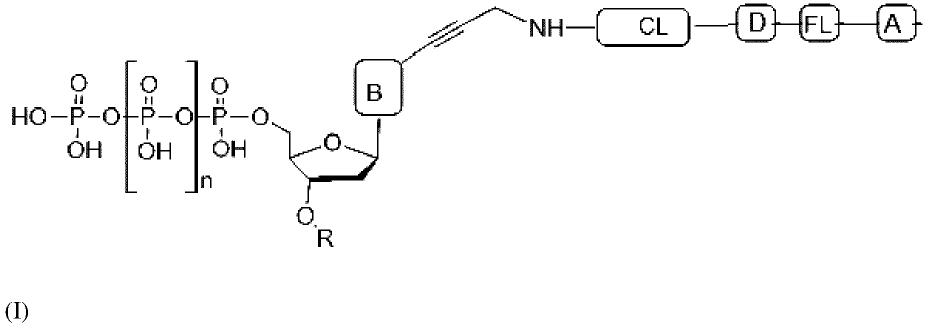 wherein B is a nucleobase
R is a reversible capping group
n is an integer from 1 to 10
CL is a chemically cleavable linker
D is a donor fluorescent dye
A is an acceptor dye
FL is a linker group

The general preparation of deoxynucleotide triphosphates having capping groups and chemically cleavable linker groups known and disclosed for example in the following patents: (1) Nucleotide analogues, patent number US 10301346, filed: November 4, 2016, date of patent: May 28, 2019. (2) Methods of using nucleotide analogues, patent number: US 10273539, filed: November 4, 2016, date of patent: April 30, 2019, (3) Methods for synthesizing nucleotide analogues with disulfide linkers, patent number: US 10336785, filed: November 4, 2016, date of patent: July 2, 2019.

In those patents, a new class of reversible nucleotide terminator is disclosed, where the 3'OH is capped with methyl-methylenedisulfide group (-CH₂SSMe) and cleavable linker with similar functional moiety (-OCH₂SS-C(Me)₂-, ref patent no. US 10301346.

This linker, which after chemical cleavage does not leave any sulfur trace, contrary to standard disulfide bonds. The capping group (-CH₂SSMe) is small, yet stable, which is essential for enzymatic incorporation and effective termination. Because of this functional group presence in nucleotide, it temporarily stops DNA synthesis by just single base incorporation. The cleavable linker which has similar functional group as the capping group, -OCH₂SS-C(Me)₂-, allowed us one step, single reaction removal of both the capping group and the fluorescent dyes.

To overcome some of the inherent limitations of the of reversible nucleotide terminators disclosed here, we have come up with the idea of using FRET dyes to label the reversible nucleotides. All four nucleotides can be labeled by separate pair of FRET dyes, or in combination with singly labeled nucleotide analogues.

FRET dyes are used in protein structure studies and in Sanger based nucleic acid sequencing. In the Sanger based sequencing, the dideoxy nucleotides are used which permanently terminate DNA synthesis and are amenable only for solution based sequencing. The randomly terminated DNA fragments are separated based on lengths by a method widely known as electrophoresis and nucleotide sequence are obtained from the relative mobility of the fragment nucleic acids. Currently, the most extensively used next-generation DNA sequencing technologies rely on reversible nucleotide terminators and sequencing takes place on solid surface. With the transition to solid phase DNA sequencing, where reversible nucleotides are used, it might be possible to use such FRET labeling on reversible nucleotides terminators. Here we demonstrate use of FRET dye label on reversible nucleotide termination, their nucleotide construct, composition and their uses are described herein.

A further object of the invention is a method for analyzing a DNA sequence on solid bound DNA templates comprised of the steps of
a) allowing nucleic acid templates with a common primer to be hybridized to said template forming a primer/template hybridization complex,
b) adding DNA polymerase, and at least one deoxynucleoside triphosphate according to any of the claims 1 to 5 and subjecting said reaction mixture to conditions so that DNA polymerase can catalyze primer extension reaction and terminate by single base extension.
c) detecting of the label nucleotides by exciting with a specific light and imaging specific to the acceptor dye's emission.

Preferable, the method is performed in that a mixture of deoxynucleoside triphosphates according to the invention is provided to the solid bond DNA templated wherein the mixture contains at least 4 different deoxynucleoside triphosphates each comprising one of the nucleotides A, T, G, C.

### BRIEF DESCRIPTION OF THE DRAWINGS

Figure 1 shows :(a) generic structure of FRET labeled reversible nucleotide terminators, where B can be natural or non-natural modified bases, (b) specific example of FRET dye labeled, A nucleotide.

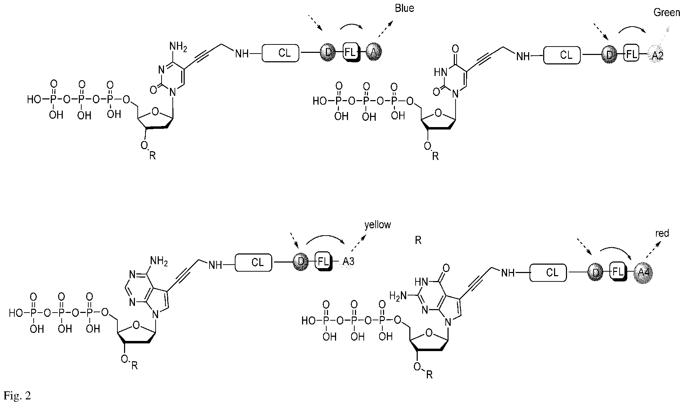

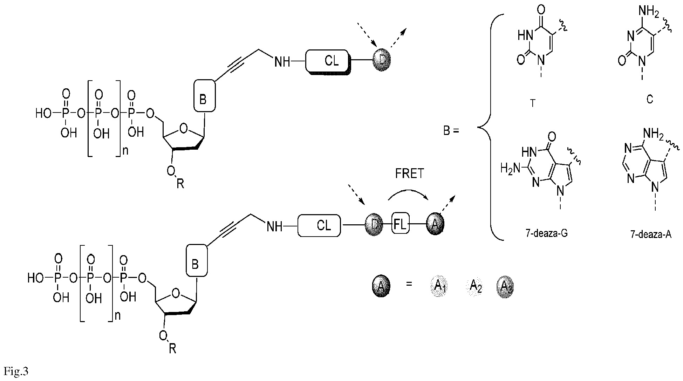

Figure 2 shows cleavable linker that connect FRET dyes to modified nucleobases. It can be cleaved chemically under the same condition used to cleave the 3'-OH capping group or in two separate steps chemical reactions.
Figure 4 shows four reversibly terminating nucleotides differently labeled with FRET dyes, while Figure 5 - 7 show how sets of differently FRET dye labeling and the single dye labeling could be used in combination to excite all four nucleobases using only one or two laser sources, in contrary to four lasers for four nucleobases in traditional sequencers.
Figure 7 shows base calling of three consecutive nucleotides - AGC, where A nucleotide was labeled by FRET dye and excited by its donor Alexa 488 specific wavelength.
Figure 8 shows a wider view of image of the DNA sequencing using mixture of FRET dye labeled nucleotide (A) and single dye labeled nucleotides (C, T and G). The yellow represents base specific incorporation of FRET labeled A, base calling for complement T base in DNA
Figure 9 to 11 show reaction schemes of the examples
Figure 12 shown preferred deoxynucleoside triphosphates according to the invention.

### DETAILED DESCRIPTION

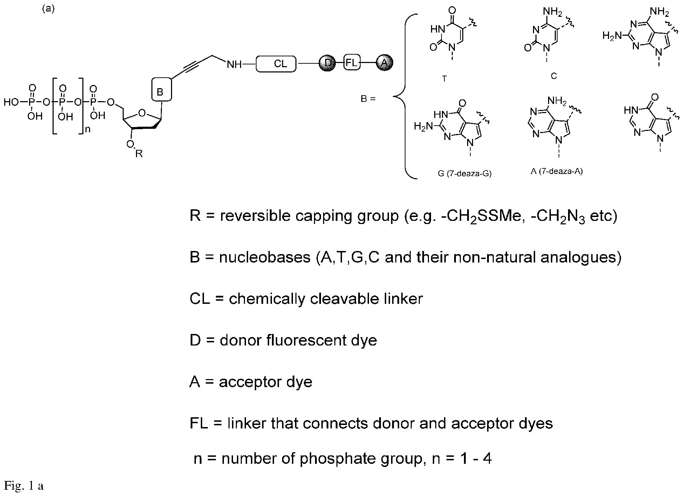

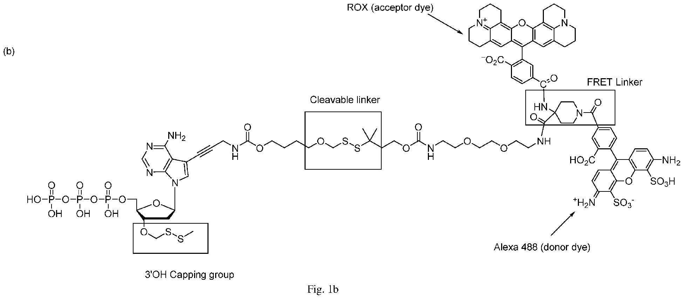

The generic structure of the FRET dye labeled nucleotide disclosed herein is shown in Figure 1. B represents nucleobases, it can be pyrimidine (T,C) or purines (A,G) or their modified non-natural analogues (e.g. 7-deaza purines, deaminated purines, etc).

The R group is reversible capping group such as -CH₂SSMe, -CH₂N₃, which can stop chain elongation by single base extension and can be removed by chemical treatment under DNA compatible conditions.

CL is cleavable linker that connects FRET dye to 5-position of the pyrimidine bases or 7-position of the purine bases of the nucleotides *via* a common propargyl group and/or its analogues.

The FRET dye consists of a donor dye (D) and acceptor dye (A) connected by common FRET linker (FL) that keeps the two dyes in close proximity and proper spatial orientation for effective energy transfer to happen when the donor dye is excited by a specific wavelength of light. Donor dye (D) can be identical or different for four nucleotides dyes, while acceptor dyes can be varied - different acceptor dye for different nucleobases. The base plurality can be deciphered by the emission of the acceptor dyes by exciting with specific light source characteristics to the donor dye's emission. The number n dictates whether it is triphosphate (n=1) or polyphosphate (n>1).

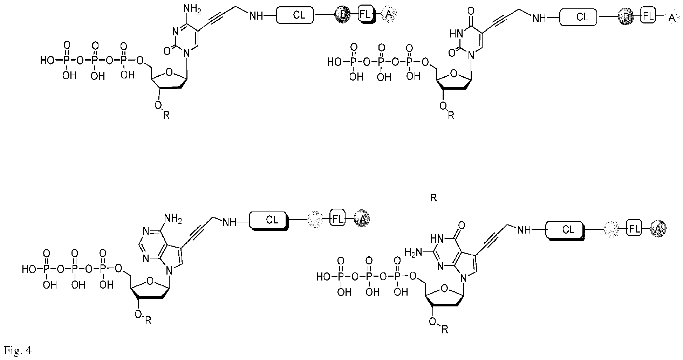

One aspect of the composition of FRET dye labeled reversible nucleotide terminators in nucleic acid sequencing can be shown in the figure 4, where all four reversible terminators have a common donor dyes but different acceptors dye. In this configuration, all four bases can be excited by exciting only the common donor dye.

Here common dye is (D), each nucleotide is combined with different acceptor fluorescent dyes (A₁, A₂, A₃, A₄) in different nucleotides. The composition of nucleotides with such construct allows excitation of the common donor fluorescent dye (D) by a specific wavelength causes energy transfer to happen to all acceptor dyes. Each nucleotide would fluorescens at different wavelength - four emission bands for four bases, despite excited by a single wavelength.

By imaging at four different filters depending on the acceptor dyes' emission light, the base calling can be possible. Here, 3'OH capping group can be, but not limited to, -methylmethylene disulfide (-CH₂SSMe), azidomethyl (-CH₂N₃), the cleavable linker can comprise of disulfide (-SS-), oxymethylene disulfide (-OCH₂-SS-), or -azidomethyl (-OCHN₃-) functional moiety.

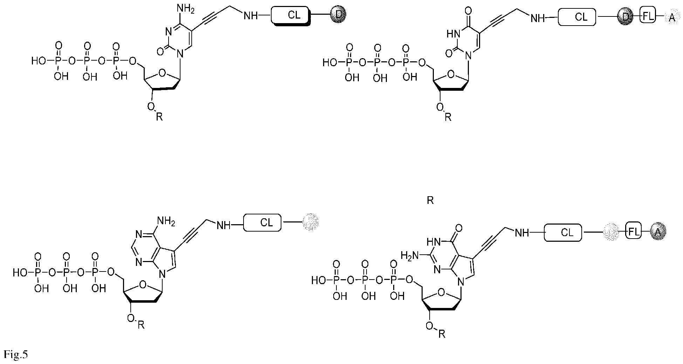

In another aspect of the invention is combination of single dye labeled reversible terminator with FRET labeled reversible terminators. Out of four labeled nucleotides, only one of them is labeled with a single fluorescent dye and the rest three bases are labeled by FRET dyes (Figure 5).

Of the rest, each of three bases is labeled by different energy transfer dye pair as shown in figure 5. Among those the donor dye is identical or similarly absorbing dye as to the singly labeled nucleotide. In this case, the emission of the single donor dye labeled nucleotide is its own emission, while the rest are from the acceptor dye's emission of the FRET pair. This combination of single dye labeled nucleotides and the FRET dyes labeled nucleotides could potentially allow nucleic acid sequencing by single laser excitation.

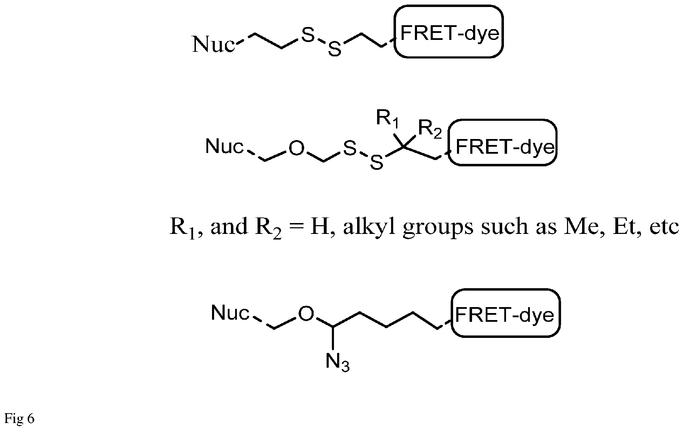

In other aspect, out of four nucleotides, two of them labeled by two different common donors and the other two by another set of pair (Figure 6). In this case, the four bases can be excited by two lasers. This configuration could allow to separate emission bands further apart from each other's, giving better resolution and thereby greater advantages in base calling.

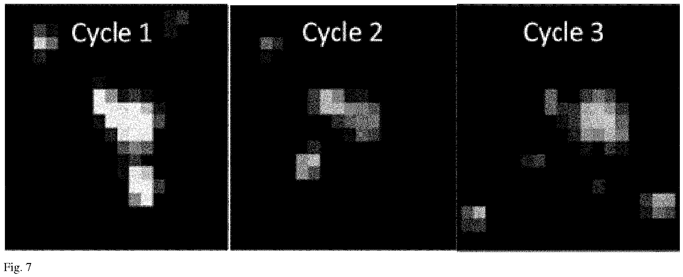

In another aspect, two of them can also be labeled by two separate single donor dyes. They are excitable at different wavelengths. The other two bases with FRET dyes consisting of different acceptor dyes, which are excitable differentially at the same wavelength as the single dyes (Figure 7). This construct could allow separation of the emission bands of the nucleotides further apart for minimizing overlap (crosstalk) of emission spectrum. This configuration potentially can help in base calling.

### Cleavable Linker (CL)

The cleavable linker that attach FRET dye pair to nucleotide's 5 - position for pyrimidines, 7 - position to purine based nucleotides, can have features that can be cleaved under the same condition as the 3'O capping group, or by separately at two different conditions.

The cleavable may comprise at least one functional group selected from the group consisting of oxymethylenedisulfide (e.g. -OCH2SSCMe2-, -OCH2SSCH2-), oxymethinetrinitride (-OCHN3-), disulfide (-SS-), or their functional analogues. Preferred variants of the cleavable linker are shown in formula (3) in Figure 6.

### 3'-OH capping group R

The capping group on 3'OH can be -CH₂SSMe, -CH₂N₃ that can be removed under DNA compatible condition after single base incorporation to allow incorporation of the subsequent base. The cleave reagents can be thiol-based cleave reagent or any other reducing agents such as TCEP or other phosphines.

### fluorescent dye D

The deoxynucleotide triphosphates according to the invention comprises a donor fluorescent dye D which may be a fluorescein dye, cyanine dye, rhodhamine dye, d-rhodamine dye, alexa dye, ATTO dye or a 5- or 6-carboxy isomer of 4'-aminomethylfluorescein according to formula (3).

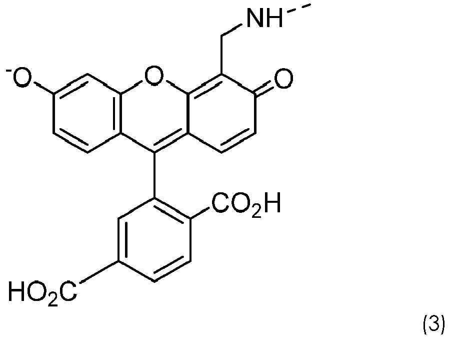

### Acceptor dye A

The deoxynucleotide triphosphates according to the invention comprises an acceptor dye A which may be a cyanine dye, rhodhamine dye, d-rhodamine dye, alexa dye, ATTO dye or their structural analogues with the absorption light overlapping with the emission light of the donor dye (D).

### Linker group FL

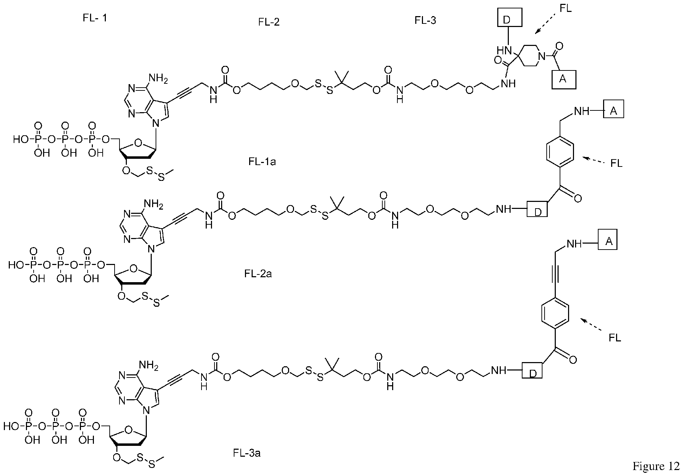

The linker group FL may be any group capable of linking the acceptor dye A and the donor dye D together to maintain appropriate distance and spatial orientation for effective energy transfer to happen when the acceptor dye is excited by specific wavelength of light. It can be trifunctional group (e.g. FL-1) capable of tethering to all - the acceptor A, the donor D and the linker CL as shown in formula (4). Preferred linker group FL are shown as FL-1, FL-2 and FL-3 which may be used to obtain the deoxynucleotide triphosphates FL-1a, FL-2a and FL-3a shown in Figure 12.

An specific example is FL-1a where the nucleotide A is attached to FL-1 which carries both the acceptor and donor dyes. It can also be bifunctional group (e.g. FL-2) capable of holding only the donor (D) and the acceptor dye (A) as shown in formula (4). An specific example is FL-2a, where the nucleotide A is attached FL-2 linker via the cleavable linker and the donor dyes in sequence. In this case one the functional groups of the donor dye is used to attach to the cleavable linker CL. Therefore, the nucleotide, the cleavable linker CL, the donor dye D, the FL linker and the acceptor dye A are arranged linearly. The other linker shown in (4), FL-3 is similar to the latter but with different functional group and therefore with a different distance from donor to acceptor dye. An specific example in FL-3a.

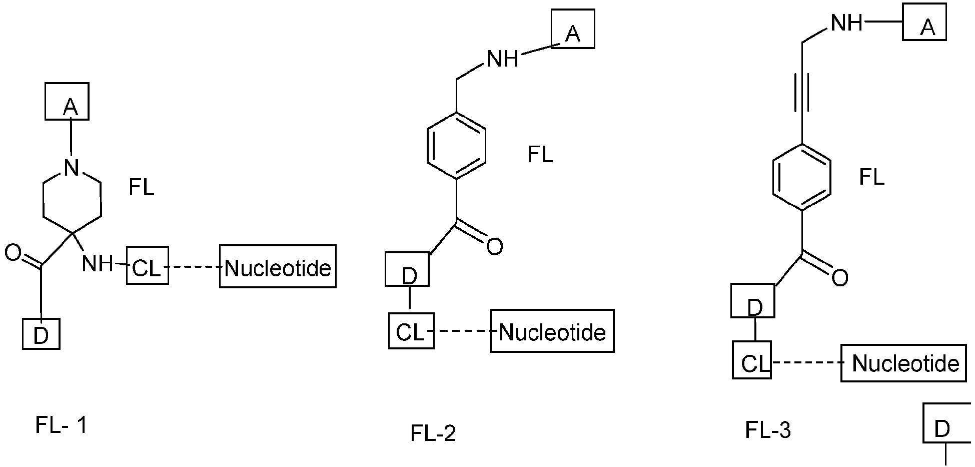

### Examples

### Synthesis of FRET dye labeled A: AF488-ROX-dATP (11, Figure 1 (b)):

As a proof of concept, one pair FRET dye was synthesized according to Scheme 1 and conjugated to reversible nucleotide terminator A. Their incorporation and base calling experiment were carried out.

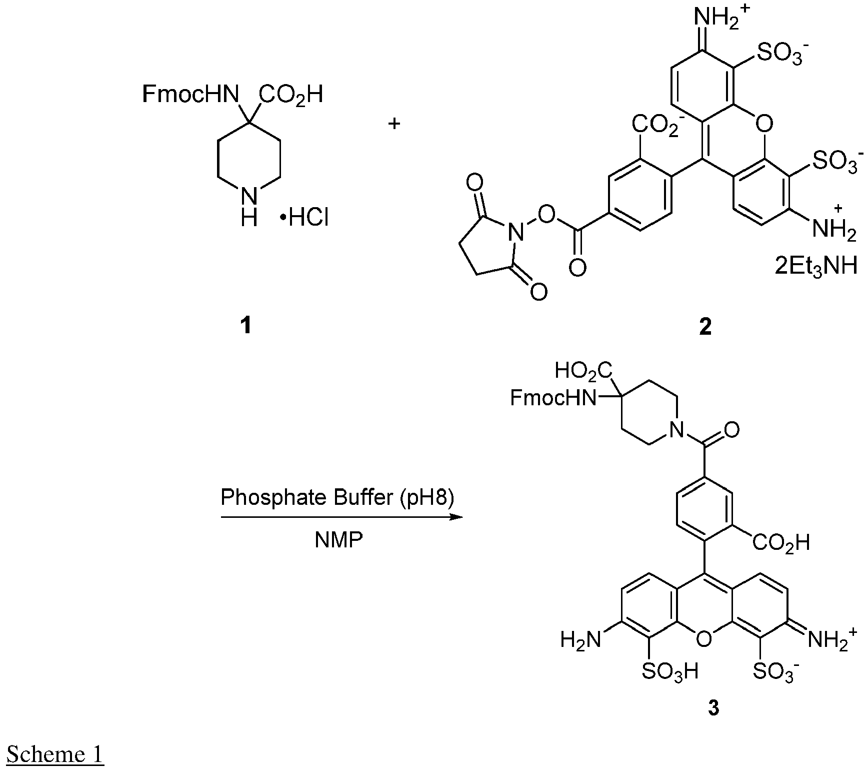

### Synthesis:

The phosphate buffer (pH 8, 500 mM, 13.3 mL) was further diluted with H₂O (31.1 mL). It was then added to a stirred solution of piperidine-4-Fmoc-OH (**1**, 40.3 mg, 100 µmol) in NMP (22.2 mL) in a water bath. The water bath was then removed, and solid AFDye488-NHS (167 mg, 200 µmol, 2 *eq.*) was then added in portion-wise and the resulting reaction mixture was stirred at room temperature for 1.0 hour. More solid AFDye488-NHS (167 mg, 200 µmol, 2 *eq.*) was added portion-wise and the resulting reaction mixture was stirred at room temperature for another 3 hrs. The completion of the dye coupling was confirmed by UPLC. The mixture was purified by flash C18 chromatography (column: 275 g RediSep Rf HP C18Aq gold column; Gradient: 0 to 2 min 100%A, then 40%B over 50 mins, A = 100 mM TEAB in H₂O, B = ACN; Flow rate: 125 mL/min.). The combined product fractions were lyophilized to dryness. The residue was dissolved in H₂O & combined to give the desired product **3** solution (10 mL, 6.6 mM *(by UV-spectrometry,* 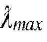 = 495 *nm, Eₘₐₓ* = *71,000 Lmol⁻¹cm⁻¹*)*,* total yield: 66 µmol, 66%).

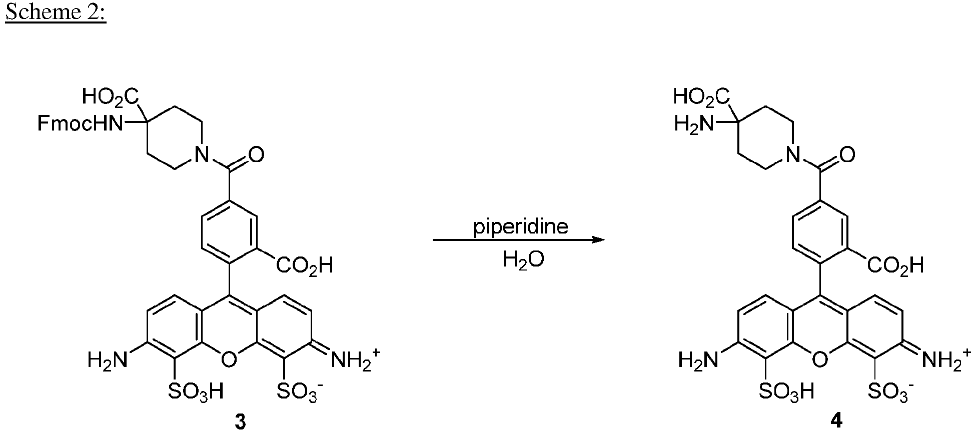

The aqueous solution of intermediate **3** was further diluted with H₂O to 30 mL, followed by the addition of piperidine (3 mL, 10% v/v, scheme 2). The resulting reaction mixture was stirred at room temperature for one hour. The completion of deprotecting was confirmed by UPLC. The mixture was purified immediately by C18 chromatography (column: 275 g RediSep Rf HP C18Aq Gold column; Gradient: 0 to 5 min 100%A, then 15%B over 50 mins, A = 100 mM TEAB in H₂O, B = ACN; flow rate: 125 mL/min.). The product fractions were lyophilized to dryness and the solid residue was dissolved in water and combined to afford the desire product **4** solution (10 mL, 5.08 mM (by UV-spectrometry, 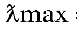 = 495 nm, *Emax* = 71,000 Lmol⁻¹cm⁻¹), total yield: 52 µmol, 77%).

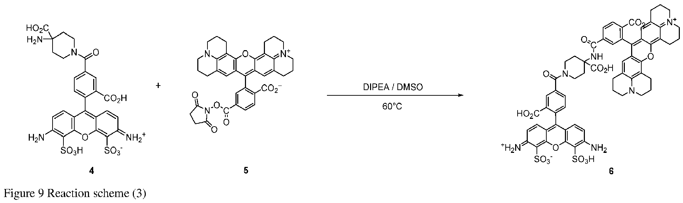

To a solution of compound **4** in anhydrous DMSO (1.25 mL, 8 mM, 10 µmol) in a 1.5 mL vial was added DIPEA (125 µL), followed by the addition of solid ROX-NHS ester 5 (12.7 mg, 20 µmol, 2 eq.). The resulting reaction mixture was heated to 60°C and shaken for 5 hours. More ROX-NHS ester (compound **5,** 19 mg, 30 µmol, 3 eq.) was added and the resulting reaction mixture was heated at 60°C and shaken overnight (scheme 3, Figure 9). The mixture was then purified by C18 Prep-LC (column: SunFire^{®} Prep C18 OBD^{™} 5µm 30x250 mm; Gradient: 0 to 2 min 100%A, then 40%B over 45 mins, A = 50 mM TEAB in H₂O, B = ACN; Flow rate: 30 mL/min.). The combined product fractions were lyophilized to dryness. The residue was dissolved in anhydrous DMSO & combined to give the desired product **6** solution (300 µL, 9.1 mM (by UV-spectrometry, 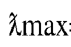 = 580 nm, *Emax* = 82,000 Lmol⁻¹cm⁻¹), total yield: 2.7 µmol, 27%). The reaction scheme (3) is shown in Fig. 9

To a solution of **4** in anhydrous DMSO (1.0 mL, 1 mM, 1.0 µmol) in a 1.5 mL vial was added DIPEA (100 µL), followed by the addition of solid AFDye 532-NHS ester (compound 7, 2.9 mg, 4 µmol, 4 *eq.).* The resulting reaction mixture was heated to 60°C and shaken for 3 hours. More ROX-NHS ester **5** (2.1 mg, 2.9 µmol, 2.9 *eq.)* was added and the resulting reaction mixture was heated at 60 °C and shaken overnight. The mixture was then purified by C18 Prep-LC (column: SunFire^{®} Prep C18 OBD^{™} 5µm 30x250 mm; Gradient: 0 to 5 min 100%A, then 20%B over 55 mins, A = 50 mM TEAB in H₂O, B = 50 mM TEAB in ACN; Flow rate: 30 mL/min.). The combined product fractions were lyophilized to dryness. The residue was dissolved in anhydrous H₂O & combined to give the desired product **8** solution (100 µL, 0.11 mM *(by UV-spectrometry,* 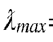 = 580 *nm, Eₘₐₓ*= *82,000 Lmol⁻¹cm⁻¹*)*,* total yield: 11.0 nmol).

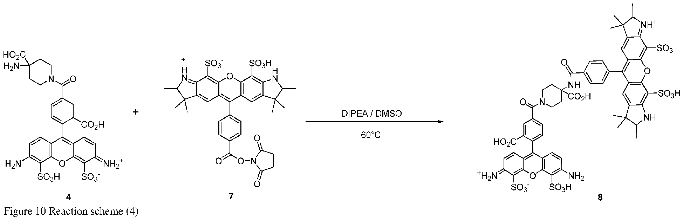

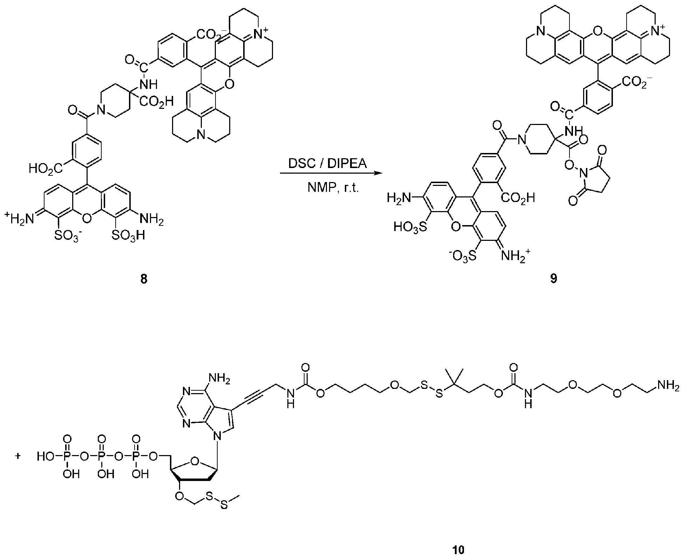

The reaction scheme (4) is shown in Fig. 10

To a solution of **8** (0.2 µmol) in anhydrous NMP (100 µL) in a 5 mL Eppendorf vial, was added a freshly prepared solution of DSC & DIPEA in anhydrous NMP (30 µL, 10 mM, 0.3 µmol, 1.5 *eq.).* The reaction mixture was shaken at room temperature for 2 hours. A solution of MeSSdATP-ARA-NH₂ **10** (0.6 µmol, 3 *eq.)* and Bu₃N (2.4 µL) in 50 µL of anhydrous NMP was added at once. The resulting RXN mixture was heated to at 60°C and shaken for 4 hours. After cooling to room temperature, the reaction mixture was diluted with TEAB buffer (6 mL, 100 mM) and purified C18 Prep-LC (column: SunFire^{®} Prep C18 OBD^{™} 5µm 19x250 mm; Gradient: 0 to 2 min 100%A, then 40%B over 55 mins, A = 50 mM TEAB in H₂O, B = 50 mM TEAB in ACN; Flow rate: 20 mL/min.). The product fraction was lyophilized to dryness. The residue was dissolved in anhydrous TE (1x) to give the desired product **11** solution (750 µL, 0.49 mM *(by UV-spectrometry,* 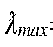 = 532 *nm, Eₘₐₓ*= *81,000 Lmol⁻¹cm⁻¹*)*,* total yield: 0.37 µmol, 37%), confirmed by HPLC-HRMS (ESI): *mlz* calcd. for C₉₄H₁₀₈N₁₃O₃₄P₃S₆: 2248.4692 [M]; Found: 1123.2334 [M⁻²]²⁻.

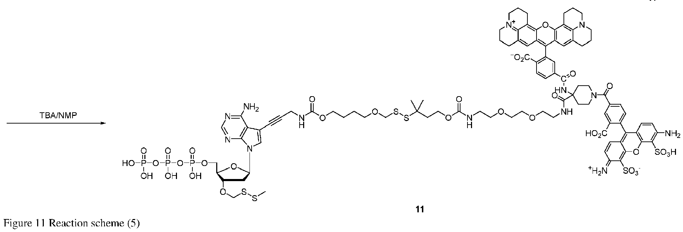

The reaction scheme (5) is shown in Fig. 11.

### Sequence specific incorporation and base calling using FRET coupled labeled A (11):

For analysis of the sequence a stretch of DNA, the DNA library sample undergoes multiple cycles of polymerase extension, fluorescent detection, and cleavage. Here we demonstrate three base-specific incorporation and imaging using one FRET labeled nucleotide (compound **11**) and the rest three nucleotides were single dye labeled (C nucleotide labeled by AF488 dye, T nucleotide labeled by R₆G dye, and G nucleotide labeled by Cy5 dye). The color of the FRET labeled nucleotide was yellow, excited by laser specific to its donor dye (AF488). All four bases were deciphered by only two laser lights - C, T and A nucleotide excited by 465/30nm while G by 615/45nm laser, where the A was called by excitation of its donor dyes - AF488 dye.

DNA library sample (rolonies) preparation consists of circular template preparation, circularized DNA clean up and rolling circle amplification (RCA). Single stranded circular DNA is generated by heat denaturation of double stranded library and followed by annealing with bridge guide oligo. Clean up with ExoI/III is then followed by purification. Secondary structure designed into adapter allows RCA product to fold into a ball-like rolony structure. Clonally amplified RCA nanoballs are seeded onto the surface of a flow-cell to be sequenced. Four cleavable fluorescent nucleotide mixture in polymerase extension reaction has concentration of 83.2 uM (AF488, C), 55.5 uM (R6G, T), 36 uM (AF488-ROX, A) and 29.6 uM (Cy5, G) respectively.

The instrument for fluorescent detection consists of a microscope with blue and red high-power LEDs (Luminus), an objective (Nikon NA=0.75, 20x) and a CCD camera (FLI Hyperion). The blue LED with a filter of 465/30nm and optical power of 300mW is used to excite Alexa488, R6G and Alexa488-ROX while the red LED with a filter of 615/45nm and optical power of 250mW is used as excitation of Cy5. Fluorescent signals are detected through emission filters of 520/28nm (AF488, C), 558/20nm (R6G, T), 628/40nm (AF488-ROX, A) and 670/30nm (Cy5, G) with 200ms imaging time.

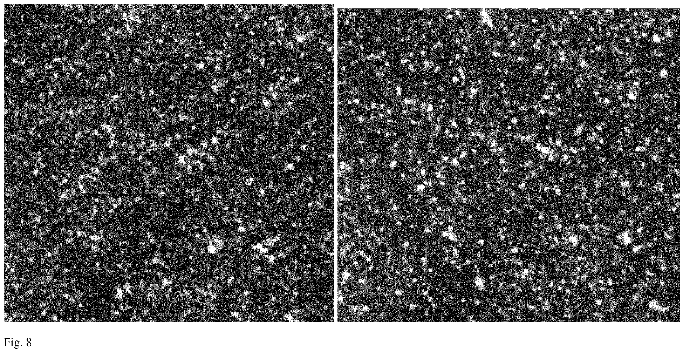

Fluorescent signals are analyzed with in house developed software. Images undergo intensity normalization, registration, cluster detection and then base-calling. Figure 8 & 9 below show a DNA rolony with changing base at different cycles.

## Claims

1. Deoxynucleotide triphosphate comprising a FRET energy transfer dye pair according to formula (I) 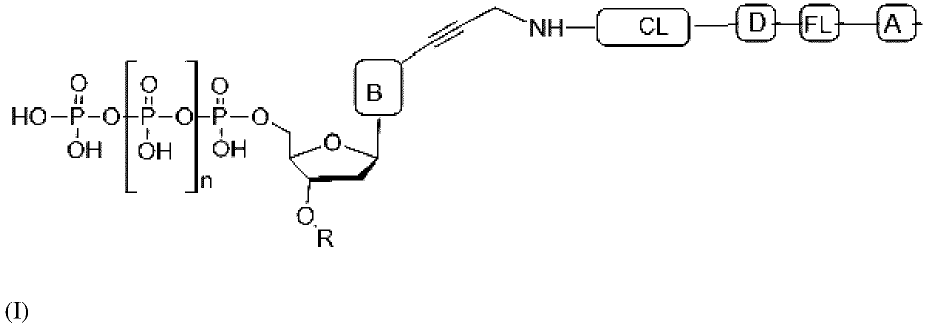 wherein B is a nucleobase
R is a reversible capping group
n is an integer from 1 to 10
CL is a chemically cleavable linker
D is a donor fluorescent dye
A is an acceptor dye
FL is a linker group

2. Deoxynucleotide triphosphate according to claim 1 **characterized in that** the reversible capping group R comprises contains at least one functional group selected from the group consisting of methylmethylenedisulfide (-CH2SSMe), azidomethyl (-CH2N3).

3. Deoxynucleotide triphosphate according to claim 1 or 2 **characterized in that** the cleavable linker (CL) contains at least one functional group selected from the group consisting of oxymethylenedisulfide (e.g. -OCH₂SSCMe₂-, -OCH₂SSCH₂-), oxymethinetrinitride (-OCHN₃-), disulfide (-SS-), or their functional analogues

4. Deoxynucleotide triphosphate according to any of the claims 1 to 3 **characterized in that** the donor fluorescent dye D is a fluorescein dye, cyanine dye, rhodhamine dye, d-rhodamine dye, alexa dye, ATTO dye or a 5- or 6-carboxy isomer of 4'-aminomethylfluorescein according to formula (3).

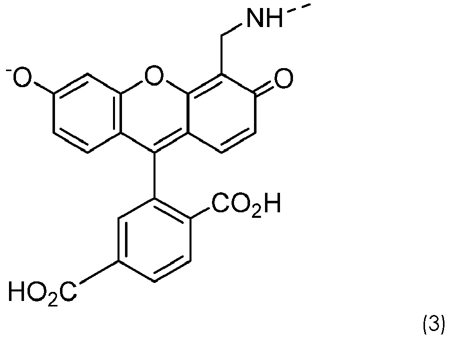

5. Deoxynucleotide triphosphate according to any of the claims 1 to 4 **characterized in that** the acceptor dye A is a cyanine dye, rhodhamine dye, d-rhodamine dye, alexa dye, ATTO dye or their structural analogues with the absorption light overlapping with the emission light of the donor dye (D).

6. A method for analyzing a DNA sequence on solid bound DNA templates comprised of the steps of
d) allowing nucleic acid templates with a common primer to be hybridized to said template forming a primer/template hybridization complex,
e) adding DNA polymerase, and at least one deoxynucleoside triphosphate according to any of the claims 1 to 5 and subjecting said reaction mixture to conditions so that DNA polymerase can catalyze primer extension reaction and terminate by single base extension.
f) detecting of the label nucleotides by exciting with a specific light and imaging specific to the acceptor dye's emission.

7. A method for analyzing a DNA sequence according to claim 6 **characterized in that** a mixture of deoxynucleoside triphosphates according to any of the claims 1 to 5 is provided to the solid bond DNA templated wherein the mixture contains at least 4 different deoxynucleoside triphosphates each comprising one of the nucleotides A, T, G, C.
